# EUROPEAN PATENT APPLICATION

(11) **EP 4 406 530 A1**
(43) Date of publication of application: **31.07.2024**
(21) Application number: 23305086.3
(22) Date of filing: 24.01.2023
(51) Int. Cl.: A61K 9/00, A61K 31/165, A61K 45/06, A61K 47/34, A61P 19/02, A61P 29/00

(54) **DOSAGE FORM FOR INTRA-ARTICULAR INJECTION COMPRISING COLCHICINE FOR USE IN THE TREATMENT OF A JOINT DISEASE SUCH AS OSTEOARTHRITIS**

(71) Applicant: PK MED, 75013 Paris (FR)
(72) Inventor: The designation of the inventor has not yet been filed
(74) Representative: Cabinet Nony

(57) **Abstract**

The present invention relates to a pharmaceutical composition under the form of a sterile and injectable dosage form comprising a controlled release dosage form comprising colchicine for use in the treatment of a joint disease such as osteoarthritis and any other type of chondropathy and/or a pain-associated joint disease, by intra-articular injection into a joint of said pharmaceutical composition, wherein the time required to release 80% by weight of the colchicine is greater than one month and wherein the colchicine is present in a concentration ranging from 0.003 to 27.5 mg per ml of the sterile and injectable dosage form. It further relates to a pharmaceutical composition under the form of a sterile and injectable dosage form comprising a controlled release dosage form comprising colchicine having an adapted *in vitro* dissolution profile. It further relates to a formulation under the form of a powder and to a pharmaceutical composition under the form of a sterile and injectable dosage form suitable for an intra-articular injection comprising colchicine, under the form of microparticles comprising a polymeric matrix and to an associated kit or article of manufacture.

## Description

The present invention relates to the field of the treatment of a joint disease such as osteoarthritis and any other type of chondropathy and/or a pain-associated joint disease in joints by colchicine. Colchicine is administered locally by intra-articular injection *via* a controlled release dosage form.

### BACKGROUND

Osteoarthritis (OA), which is in particular characterized by a chronic pain, is a multifactorial degenerative disorder of joints leading to cartilage and tissue destruction along with subchondral bone modifications and low grade of synovial inflammation. OA is very common and often an age-related or a trauma-related disease but with heterogenous phenotypes (Genetic, inflammatory, Metabolic, Senescent...). Synovitis or a low-grade local inflammation is a common feature of OA and may be at some point the driver of disease progression. More specifically calcium crystals (calcium phosphate and pyrophosphate) frequently present in OA joints may contribute to the joint inflammation.

Treatments for osteoarthritis are most often symptomatic and aimed at pain control but provide a moderate effectiveness along with safety concerns. Generally, the first line options are mainly oral nonsteroidal anti-inflammatory drugs (NSAIDs), oral or intra-articular corticosteroids. When it is possible and when the joints are accessible, i.e. in the case of large joints (knees, ankles, wrists, elbows and shoulders), intra-articular corticosteroid injection is considered by practitioners to avoid systemic administration and thus avoid the risk of significant side effects.

Intra-articular administration of corticosteroid, used for several decades in joint diseases in general, permits a slight improvement in pain and mobility, with non-lasting effects (a few weeks in the case of an immediate release form). However, the toxicological aspect of these administrations is quite problematic and also controversial. Indeed, systemic effects are mentioned for weeks following the injection including Cushing's syndrome, hyperglycemia, decreased bone density or a risk of infections. The latter being the consequence of the direct introduction of an infectious agent into the articulation or by reducing the immune response and allowing a latent tuberculosis infection to be reactivated. This type of treatment is in particular not adapted for patients at risk, i.e. for example with comorbidities, like diabetes or osteoporosis. In addition, deleterious effects are also described on local tissue as damage to articular cartilage and tendons.

Consequently, in medical practice, the frequency and dose of intra-articular corticosteroid injections are limited. For the immediate release galenic forms it is therefore recommended a maximum of three injections per year/per site and a minimum interval of three months between two injections. A first intra-articular sustained-release formulation of corticosteroid (Zilretta^{®} by Flexion Therapeutics) was approved in 2017 by the FDA for osteoarthritis and associated pain, in particular knee osteoarthritis, allowing continuous treatment for months. However, this product is not approved for repeated administrations.

Intra-articular administration of corticosteroids therefore does not seem to be an optimal treatment for chronic treatment of osteoarthritis.

Colchicine is a drug characterized by pleiotropic effects related to its capacity to bind to tubulins and disturb microtubule polymerization, destabilizing the cytoskeleton and all the cellular processes, including cell division, migration and cell shape. Among these effects, colchicine affects the immune system and downregulates multiple inflammatory pathways, leading to a decrease in neutrophil function and migration. Colchicine is therefore used and approved in several inflammatory diseases such as Familial Mediterranean Fever, Behçet's disease and Microcrystalline diseases.

Colchicine has indeed been used for a very long time for the non-specific treatment of crystal-associated arthropathies. In the past 50 years, the use of short courses of fast-acting NSAIDs and corticoids have been considered with colchicine as the oral drugs of choice for the symptomatic treatment of acute gout and pseudogout. In the article George Nuki, MB, FRCP "Colchicine: its Mechanism of Action and Efficacy in Crystal-Induced Inflammation", Current Rheumatology Reports, 2008, 10:218-227, prophylactic and therapeutic efficacy of oral administration of colchicine is reviewed. Still in said article, side effects of colchicine are extensively described, such as powerful purgative effects, bone marrow suppression, neuromyopathy and rhabdomyolysis, especially in patients with renal insufficiency as well as high mortality associated with overdosage. Colchicine has thus an exceptionally narrow therapeutic index

Colchicine is currently not an approved treatment for osteoarthritis; however some clinical studies have demonstrated that oral colchicine may be helpful in the treatment of adult patients with knee osteoarthritis, including primary osteoarthritis, i.e. not associated with the deposit of calcium pyrophosphate crystals. But oral colchicine at the tested doses can cause side effects as gastrointestinal symptoms observed in some patients. (Restrepo-Escobar M et al. Revisión sistemática de la literatura sobre el tratamiento con colchicina en pacientes adultos con osteoartritis de rodilla. Rev Colomb Reumatol. 2017;24:102-111) and other reviews failed to demonstrate such a benefit (Singh, A. et al. Efficacy and safety of colchicine for the treatment of osteoarthritis: a systematic review and meta-analysis of intervention trials. Clin Rheumatol (2022)).

Moreover, although it is known to treat inflammation or osteoarthritis in joints, i.e. at site, by directly administering an active substance therein, it has been found that the active substance escapes relatively easily from said joint, thus reducing the therapeutic effect.

Colchicine encapsulation for sustained release has been reported in different carriers, such as in polymeric microspheres (Das, G. S. et al. "Colchicine Encapsulation within Poly(Ethylene Glycol)-Coated Poly(Lactic Acid)/Poly(ε-Caprolactone) Microspheres-Controlled Release Studies" Drug Delivery, 7: 7544, 129-138 (2000)). In this article, colchicine has been investigated in the treatment of other diseases, such as restenosis after angioplasty or vascular injury, and on this occasion, attempts of encapsulation of colchicine within poly(lactic acid)/poly(ε-Caprolactone) microspheres were performed for local delivery of colchicine. Another composition comprising biodegradable microparticles containing colchicine was further described seeking the treatment of the same pathology, i.e. restenosis, through intramural delivery, as reported in Gradus-Pizlo Irmina et al: "local delivery of biodegradable microcapsules containing colchicine or a colchicine analogue: effects on restenosis and implications of catheter-based drug delivery", Journal of The American College of Cardiology, vol.26, no. 6, pages 1549-1557. It clearly comes out that the therapeutic target is completely different from the one of the present invention. In addition, the results are not encouraging and point out toxicity risks. Indeed, signs of local toxicity were observed in the muscle layers overlying and fed by arteries infused with the microparticles containing colchicine or colchicine analogue but not controlled microparticles. For both disclosures, it is furthermore worth to notice that the particle size is lower than the one of the particles implemented in the present invention, as it will be apparent in the following description. In addition, no long-lasting treatment, namely greater than one month, is disclosed nor suggested.

In addition, colchicine has previously been reported in US5,747,060, again for other effects than the one as directed to in the framework of the present invention, as acting on the prolongation of the duration of the anesthetic effect produced by the local administration of an anesthetic. Colchicine is thus co-administered in example 1 of said document around the sciatic nerve with bupivacaine, under an immediate release form, i.e. directly added into an aqueous solution. Intra-articular injection is not described in said document, nor is the activity of colchicine in the treatment of osteoarthritis in joints, and even less for a sustained release.

WO2006/066419 discloses the same effect of colchicine with respect to the prolongation of its anesthetic effect and namely a combination of a vanilloid receptor agonist and another molecule, possibly colchicine. An example discloses a composition comprising a mixture of resiniferatoxin and colchicine injected intra-articularly for the treatment of capsulitis. Said injection is however additionally dedicated for an immediate release not seeking to control the local profile and systemic pharmacokinetics of colchicine, nor to extend its therapeutic exposure.

The use of colchicine for the treatment of crystal-associated arthropathies is well established. However, nowadays, colchicine is only commercialized under an immediate release oral dosage form, classically with a daily dosage for the treatment of the crystal-associated arthropathy around 1 mg per day. The FDA-approved dosage for the treatment of acute gout flare is 1.2 mg colchicine at the first sign of the flare followed by 0.6 mg one hour late (https://www.fda.gov/drugs/postmarket-drug-safety-information-patients-and-providers/colchicine-marketed-colcrys-information). The recommended colchicine first-line treatment for acute flare by EULAR is a loading dose of 1 mg followed 1 hour later by 0.5 mg (Richette, P. et al. 2016 updated EULAR evidence-based recommendations for the management of gout. Ann. Rheum. Dis. 76, 29-42 (2017)). However, as explained above, and illustrated herein after in more details, reaching colchicine therapeutically active dose locally is hindered by the low tolerance or high toxicity of orally administered colchicine rendering the drug prescription extremely complicated.

Colchicine was recently described in a nanoemulsion system injected into the joint of Swiss albino mice model with MSU (Monosodium Urate) crystal-induced gouty arthritis: Aboumanei et al, "intra-articular formulation of colchicine loaded nanoemulsion systems for enhanced locoregional drug delivery: In vitro characterization, 99mTc coupling and in vivo biodistribution studies", DOI: 10.1080/03639045.2021.1934865. The biodistribution pattern of ^{99m}Tc-ColNE-5 as well as ^{99m}Tc-Col solution (^{99m}TcColS) was studied. However, said disclosure is limited to an observation of a radiolabeled colchicine which is thus a different molecule from colchicine itself and within a period not exceeding 24 hours. It is well known that labelling with radionuclide like ^{99m}Tc can have a strong impact on the pattern of biodistribution of the labelled entity due to modification of its charge, lipophilicity and stability (Decristoforo et al. "The influence of chelator on the pharmacokinetics of 99mTc-labelled peptides." Q. J. NUCL. MED. 46 3 (2002): 195-205). Moreover, said article is silent on the technical problem of narrow therapeutic index as explained above and very specific to the colchicine, i.e. the maximal systemic concentration to be avoided for toxicity reasons. Said article is thus also silent on microparticulate systems with higher sizes and targeted dosages able to reach effective local concentration for combatting osteoarthritis, with no toxicity at a systemic level. It namely appears in figure 3 of said article that ColNE-5, i.e. « colchicine nanoemulsion system » provides a release of 40% of colchicine in less than 3 hours, which is contrary to the fulfilment of a controlled systemic concentration of colchicine for avoiding side effects, as required in the present invention, and wherein the time required to release 80% by weight of the colchicine is greater than one month. Achieving release profiles not having such a quick release just after the administration, also called "burst release", as explained herein after, that can be detrimental in terms of side effects in such an extent, implies as such a non-obvious approach which was not predicable from said article. In other words, no colchicine-controlled release profile suitable for human healing is revealed in said article, and even more particularly for the treatment of osteoarthritis.

IV injection of colchicine is considered effective in the treatment of acute gout attack with a dose not exceeding 2-3 mg per single injection (Nuki, G. Colchicine: Its mechanism of action and efficacy in crystal-induced inflammation. Curr. Rheumatol. Rep. 10, 218-227 (2008)). However even if GI side effects can be avoided using i.v. administration, severe toxicity was associated with inappropriate dosing (Wallace SL, S. J. Review: systemic toxicity associated with the intravenous administration of colchicineguidelines for use. J Rheumatol. 15, 495-9 (1988), Bonnel, R. A., Villalba, M. L., Karwoski, C. B. & Beitz, J. Deaths associated with inappropriate intravenous colchicine administration. J. Emerg. Med. 22, 385-387 (2002)). This led authorities to eliminate the use of i.v. colchicine for acute gout treatment (Zhang, W. et al. EULAR evidence-based recommendations for gout. Part II: Management. Report of a task force of the EULAR Standing Committee for International Clinical Studies Including Therapeutics (ESCISIT). Ann. Rheum. Dis. 65, 1312-1324 (2006)). Attempts of i.v. injection of colchicine have furthermore largely discouraged the man skilled in the art to envision injection of colchicine, as reported in E.Niel et al, "Colchicine today", Joint Bone Spine 73 (2006) 672-678.

Therefore, it remains a need to develop novel dosage forms, route of administrations and targeted dosage for colchicine, both for obviating the side-effects, in particular observed during systemic administration, and improving the efficacy by optimizing the available dose of colchicine, in particular for extending its local duration of action at the painful site, in particular by increasing the local concentration, for the treatment of a joint disease such as osteoarthritis and any other type of chondropathy and/or a pain-associated joint disease, in particular osteoarthritis and/or pain-associated osteoarthritis.

The invention has for purpose to meet the needs, thereby both obtaining increased advantage from its action against the pain and/or destruction of the joint cartilage of patients subject to a joint disease such as osteoarthritis and any other type of chondropathy and/or a pain-associated joint disease, such as osteoarthritis and any other type of chondropathy and/or a pain-associated joint disease, in particular osteoarthritis, and restricting the level of any side effects, including of osteoarthritis patients with inflammatory component, inflammatory pain or joint effusion.

One of the advantages of the present invention is to provide a new mean to achieve a particular colchicine release profile which respects an adequate ratio between the local concentration of colchicine and the systemic concentration thereof, suitable for offering the best therapeutic action locally and at the same time avoiding potential systemic toxicity while providing pain relief and inflammation decrease immediately and throughout the duration of the treatment than can last more than one month, for example between one and six months, with only one intra-articular injection, that can be renewed.

Due to the reduced side-effects obtained by the administration of the pharmaceutical composition according to the present invention, it is furthermore particularly suitable for people suffering from chronic comorbidities (Hypertension, Chronic Kidney diseases, Diabete, Heart diseases...) fairly prevalent conditions in osteoarthritis. Indeed, the management of osteoarthritis patients having multiple comorbidities, remains a challenge for medical practitioners due to frequent and major contraindications to either NSAIDs or Corticosteroids. In comparison to the existing oral colchicine treatment, the present invention, in addition to avoid gastrointestinal side effects, prevents major toxicity risks for patients suffering from renal or hepatic impairments or for co-medicated patients with CYP3A4/PGP drug inhibitors, which are fairly prevalent medications.

### SUMMARY OF THE INVENTION

According to a first aspect, herein is provided a pharmaceutical composition under the form of a sterile and injectable dosage form comprising a controlled release dosage form comprising colchicine for use in the treatment of a joint disease such as osteoarthritis and any other type of chondropathy and/or a pain-associated joint disease, by intra-articular injection into a joint of said pharmaceutical composition, wherein the time required to release 80% by weight of the colchicine is greater than one month and wherein the colchicine is present in a concentration ranging from 0.003 to 27.5 mg per ml of the sterile and injectable dosage form.

According to a second aspect, herein is provided a pharmaceutical composition under the form of a sterile and injectable dosage form comprising a controlled release dosage form comprising colchicine, wherein said pharmaceutical composition presents an *in vitro* dissolution profile, wherein less than 25% of colchicine, and more preferentially less than 15% of colchicine, is released within 24 hours, more than 80% of colchicine is released within at least one month, in particular within three months, and even more particularly within six months, and wherein the colchicine is present in a concentration ranging from 0.003 to 27.5 mg per ml of the sterile and injectable dosage form.

According to a third aspect, herein is provided a formulation under the form of a powder comprising a controlled release dosage form comprising colchicine, under the form of microparticles having a mean particle size determined by laser light diffraction measurement method equal or greater than 10 µm, comprising a polymeric matrix, wherein the polymeric matrix comprises at least one poly(lactic-co-glycolic acid) copolymer, at least one poly(caprolactone), or a mixture of at least one poly(lactic-co-glycolic acid) copolymer and at least one poly(caprolactone) in an amount of greater than 70% by weight, with respect to the total weight of the polymeric matrix, in particular greater than 80% by weight, and even more particularly greater than 90% by weight, wherein the poly(lactic-co-glycolic acid) copolymer has in particular a lactic:glycolic molar ratio ranging between 50:50 and 90:10, in particular ranging between 60:40 and 90:10, more particularly ranging between 65:35 and 85:15, for example 75:25, and wherein the poly(lactic-co-glycolic acid) copolymer in particular exhibits an inherent viscosity determined by an Ubbelohde capillary viscometer of 0.1 to 1.7 dl/g, in particular of 0.1 to 1.4 dl/g and even more particularly from 0.1 to 0.9 dl/g.

According to a fourth aspect, herein is provided a pharmaceutical composition under the form of a sterile and injectable dosage form suitable for an intra-articular injection, obtained by mixing a formulation under the form of a powder according to the present invention, with an aqueous injection vehicle, wherein the pharmaceutical composition optionally comprises an excipient selected from a group consisting of a tonicity-enhancing agent, a wetting-agent, a viscosity-enhancing agent, a density-enhancing agent or a mixture thereof, and wherein said excipient may be present in the aqueous injection vehicle or in the powder.

According to a fifth aspect, herein is provided a pharmaceutical composition under the form of a sterile and injectable dosage form suitable for an intra-articular injection, comprising a controlled release dosage form comprising colchicine, under the form of microparticles having a mean particle size determined by laser light diffraction measurement method equal or greater than 10 µm comprising a polymeric matrix, wherein the polymeric matrix comprises one at least poly(lactic-co-glycolic acid) copolymer, at least one poly(caprolactone), or a mixture of at least one poly(lactic-co-glycolic acid) copolymer and at least one poly(caprolactone) in an amount of greater than 70% by weight, with respect to the total weight of the polymeric matrix, in particular greater than 80% by weight, and even more particularly greater than 90% by weight, wherein the poly(lactic-co-glycolic acid) copolymer has in particular a lactic:glycolic molar ratio ranging between 50:50 and 90:10, in particular ranging between 60:40 and 90:10, more particularly ranging between 65:35 and 85:15, for example 75:25, and wherein the poly(lactic-co-glycolic acid) copolymer in particular exhibits an inherent viscosity determined by an Ubbelohde capillary viscometer of 0.1 to 1.7 dl/g, in particular of 0.1 to 1.4 dl/g and even more particularly from 0.1 to 0.9 dl/g.

According to a sixth aspect, herein is provided a kit or article of manufacture comprising, in separate compartments (i) an aqueous injection vehicle and (ii) a controlled release dosage form comprising colchicine as defined hereinafter or powder as defined herein after, wherein the kit or article of manufacture optionally comprises an excipient selected from a group consisting of a tonicity-enhancing agent, a wetting-agent, a viscosity-enhancing agent, a density-enhancing agent or a mixture thereof, for preparing a pharmaceutical composition suitable for an intra-articular injection.

The pharmaceutical composition suitable for an intra-articular injection as described herein after is effective in treating a joint disease such as osteoarthritis and any other type of chondropathy and/or a pain-associated joint disease, particular osteoarthritis and/or a pain-associated osteoarthritis, with minimal long-term side effects.

Said pharmaceutical composition suitable for an intra-articular injection is suitable for local administration by injection into a site at or near the site of pain.

### DETAILED DESCRIPTION OF THE INVENTION

As apparent from the BACKGROUND paragraph above, the prescription of colchicine is nowadays delicate as only oral compositions are available and high risk of toxicity have been observed, such as gastrointestinal symptoms and neuromuscular involvement. Risks linked to interaction with other active agents have further been reported. And again, toxicity constraints frankly have discouraged the local injection for decades even though the use of colchicine has sometimes been described as encouraging for treating osteoarthritis.

Indeed, it has been established in the literature that effective local dose of 0.015 mg/kg to 0.030 mg/kg colchicine is recommended, that at 0.1 mg/kg the toxicity limit is reached, and that at 0.8 mg/kg lethal dose is reached (E.Niel et al, "Colchicine today", Joint Bone Spine 73 (2006) 672-678). It is thus concluded that the narrow therapeutic margin for colchicine is a source of concern for prescribing physicians.

The inventors have found that compared to the treatment of oral colchicine or intra-articular corticosteroids in joint pain diseases, the intra-articular injection of a pharmaceutical composition according to the present invention, for a controlled release of colchicine has at least equivalent and even higher efficiency.

It provides joint disease treatment, and in particular osteoarthritis and any other type of chondropathy and/or a pain-associated joint disease, more particularly osteoarthritis treatment and/or better pain relief as soon as administered and during the release of the colchicine. In this invention, the time required to release 80% by weight of the colchicine, in particular from the controlled release dosage form comprising it, and more particularly from the microparticles, is greater than one month, and in particular is between one month and six months, and colchicine is present in a concentration ranging from 0.003 to 27.5 mg per ml of the sterile and injectable dosage form, in particular a suspension.

Osteoarthritis not only induces cartilage degradation and associated chronic pain but also flare-ups that may be very intense. Thus, having treatments perfectly adapted for achieving the most appropriate dosage is crucial.

The use or method of treatment according to the present invention also has the advantage of reducing the significant toxicity risks, linked to drug interaction with colchicine when administered *via* a systemic route, and in particular in the elderly, or patients with kidney or liver failure as explained herein after.

In comparison to intra-articular corticosteroids, this treatment also has the advantage to avoid systemic effects such as Cushing's syndrome, hyperglycemia, hypertension, decreased bone density or even the risk of infections. In addition, it decreases the risk of deleterious effects on local tissue as damage to articular cartilage and tendons.

### Definitions

As used herein, the term "patient" refers to either an animal, such as a valuable animal for breeding, company or preservation purposes, or preferably a human or a human child, which is afflicted with, or has the potential to be afflicted with osteoarthritis.

In particular, as used in the present application, the term "patient" refers to a mammal, including a non-human mammal such as a rodent, cat, dog, or primate, or a human; preferably said subject is a human and also extends to birds.

The identification of those patients who are in need of treatment of a joint disease, such as osteoarthritis and any other type of chondropathy and/or a pain-associated joint disease, in particular osteoarthritis and/or a pain-associated osteoarthritis, including osteoarthritis patients with inflammatory component, inflammatory pain or joint effusion, is well within the ability and knowledge of one skilled in the art. A veterinarian or a physician skilled in the art can readily identify, by the use of clinical tests, physical examination, medical/family history or biological and diagnostic tests, those patients who are in need of such treatment.

In the context of the invention, the term "treating" or "treatment", as used herein, means reversing, alleviating, inhibiting the progress of a joint disease, such as osteoarthritis and any other type of chondropathy and/or a pain-associated joint disease, in particular of osteoarthritis and/or a pain-associated osteoarthritis.

As used herein, an "effective amount" refers to an amount of a compound of the present invention which is effective in reducing, eliminating, treating or controlling the symptoms of a joint disease, such as osteoarthritis and any other type of chondropathy and/or a pain-associated joint disease, in particular osteoarthritis. The term "controlling" is intended to refer to all processes wherein there may be a slowing, interrupting, arresting, or stopping of the progression of a joint disease, such as osteoarthritis and any other type of chondropathy and/or a pain-associated joint disease, in particular osteoarthritis and/or a pain-associated osteoarthritis, but does not necessarily indicate a total elimination of it or associated conditions as detailed herein after.

The term "treatment-effective amount" refers to a concentration of compound that is effective in treating a joint disease, such as osteoarthritis and any other type of chondropathy and/or a pain-associated joint disease, in particular osteoarthritis and/or a pain-associated osteoarthritis, e.g. leads to a reduction in inflammatory pain, following examination when administered after initiation of the inflammatory pain has occurred.

As used herein, the term "osteoarthritis" or "OA" includes all disorders and conditions associated with it, such as cartilage loss, associated inflammation and/or pain, and also all types, origins or groups of osteoarthritis. As mentioned above, OA is a degenerative disease, but symptoms can worsen for a while, and then improve, which is known as a flare-up or flare. Osteoarthritis includes inflammatory flare-ups, or any progression related to primary osteoarthritis, i.e., no known cause, or related to secondary osteoarthritis, i.e., caused by any disease such as chondropathies. Symptoms and/or conditions associated to OA and in particular OA flare-up may for example include increased joint pain, swelling of the affected area, reduced range of motion at the location of the joint and/or fatigue from increased pain.

As used herein, the term "chondropathy" includes any cause or disease that results in cartilage damage such as Chondromalacia, limb misalignment, Chondromatosis, Chondrocalcinosis, Gout, Hemochromatosis, Wilson's disease, Acromegaly, Ochronosis, collagen abnormalities, chronic neuropathies of any cause, Kashin-Beck disease or any inflammatory rheumatisms.

According to one embodiment, the use according to the present invention is for the treatment of osteoarthritis in patients with inflammatory component, inflammatory pain or joint effusion.

As used herein, the term "pharmaceutically acceptable" refers to those compounds, materials, excipients, compositions or dosage forms which are, within the scope of sound medical judgment, suitable for contact with the tissues of human beings and animals without excessive toxicity, irritation, allergic response or other problem complications commensurate with a reasonable benefit/risk ratio.

The term "pharmaceutically acceptable carrier, adjuvant, or vehicle" may refer to any pharmaceutically acceptable excipient, such as a non-toxic carrier, adjuvant, or vehicle that does not destroy the pharmacological activity of the compound with which it is formulated.

Within the framework of the present invention, the term "painful site" or "site of the pain" means the joint area causing pain where the product can be injected. It can be the knee joint, the hip joint, metacarpophalangeal joints, the shoulder joint, the wrist joint, the elbow joint, metatarsophalangeal joints, the ankle joint, the vertebral joint and the midfoot joints.

The terms "controlled release composition" or "controlled release microparticle" mean the composition or the microparticle allow a release into the body of a specified amount over a specified period of time of an active ingredient, namely a specific pharmacokinetic profile. The term "controlled release" encompasses all the type of releases that are modified in comparison to an immediate release. In other words, the term "controlled release" is equivalent to "modified release" and encloses extended releases as defined herein after as well as delayed release and pulse releases.

The terms "extended release", "prolonged release" and "sustained release" are considered equivalent in the framework of the present invention. This type of release means that the release is prolonged over time in comparison to an immediate release, i.e. it means that the active ingredient is released slowly over time, allowing a less frequent intake of the drug for the patient. In other words, the active ingredient is progressively released over a specific period of time, generally aiming at less side effects as well by decreasing the maximal concentration.

The term "drug loading content" means the mass ratio of the drug in the microparticles to the mass of microparticles.

The term "mean particle size" or D50 means the particle diameter in microns that splits the particle volume distribution with half above and half below this diameter.

A controlled or modified release may of course result from the combination of an immediate release and a controlled release.

The term "D10=x µm" as mainly used in the example means that 10% of the particles have a size of x µm or less.

The term "D90=y µm" as mainly used in the example means that 90% of the particles have a size of y µm or less. A controlled or modified release may of course result from the combination of an immediate release and a controlled release.

### COLCHICINE

Colchicine is an alkaloid extracted from the corm of the meadow saffron or autumn crocus (*Colchicum autumnale*). Its IUPAC name is N-[(7S)-1,2,3,10-tetramethoxy-9-oxo-6,7-dihydro-5H-benzo[a]heptalen-7-yl]acetamide.(CAS 64-86-8)

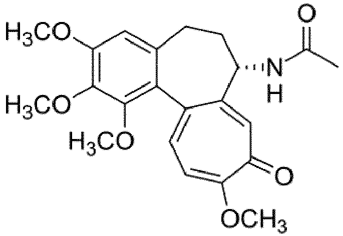

Colchicine may exist in racemic of enantiomeric form. All said forms are encompassed within the scope of the invention.

Moreover, it may exist in various crystalline forms depending on the solvent used for obtaining them. Chloroform, dichloromethane, ethyl acetate, benzene or any other suitable solvent may be cited among possible solvents. All such crystalline forms also form part of the present invention.

It is mainly known as gout suppressant and as an anti-inflammatory agent *via* its tubulin interaction activity. It is administered by the oral route although intravenous administration was also attempted and then abandoned for excessive toxicity reasons.

### THERAPEUTIC USE AND METHODS

In one embodiment, the pharmaceutical composition suitable for an intra-articular injection as described herein after delivers colchicine in a dose and in a controlled release manner such that the concentration level of colchicine at the treated articular site is above the systemic dosage classically obtained after an oral administration, i.e. above 5 nM (2 ng/mL), and the systemic concentration level stays below the value of 15 nM (6 ng/mL). (Terkeltaub, R. A. et al. High versus low dosing of oral colchicine for early acute gout flare: Twenty-four-hour outcome of the first multicenter, randomized, double-blind, placebocontrolled, parallel-group, dose-comparison colchicine study. Arthritis Rheum. 62, 1060-1068 (2010)).

According to another embodiment, the pharmaceutical composition of the invention is further characterized in that it is effective to maintain systemic concentration of colchicine 24 hours after the intra-articular injection, in particular over 1 month, and even more particularly over at least 3 months, lower than 5 ng/ml, particularly lower than 1 ng/ml, more particularly lower than 0.5 ng/ml, and even more particularly lower than 0.1 ng/ml and to maintain synovial concentration of colchicine after the intra-articular injection, over 1 month, and even more particularly over at least 3 months, greater than 0.5 ng/ml, in particular comprised between 0.5 and 100 ng/ml and more particularly comprised between 0.5 and 50 ng/ml.

The particular release profile allowing a tolerable systemic concentration of colchicine and at the same time a local concentration of colchicine suitable for having an appropriate therapeutic effect, which represents the very core of the invention, has never been obtained before. It comes out that through a particular ratio between the local or synovial concentration of colchicine and systemic concentration of colchicine obtained with the pharmaceutical composition according to the invention, i.e. with a particular *in vitro* dissolution rate and/or *in vivo* release rate, the inventors have discovered a surprisingly well adapted mean for treating the patients while avoiding side effects.

According to one particular embodiment, the ratio between the local or synovial concentration of colchicine and systemic concentration of colchicine obtained after injection into a joint of a pharmaceutical composition according to the present invention may range between 100 and 2000 in human as described/mentioned in 1) Petit, A., Redout, E. M., van de Lest, C. H., de Grauw, J. C., Müller, B., Meyboom, R., van Midwoud, P., Vermonden, T., Hennink, W. E., & René van Weeren, P. (2015). Sustained intra-articular release of celecoxib from in situ forming gels made of acetyl-capped PCLA-PEG-PCLA triblock copolymers in horses. Biomaterials, 53, 426-436. https://doi.org/10.1016/j.biomaterials.2015.02.109_and 2) Kraus VB, Conaghan PG, Aazami HA, Mehra P, Kivitz AJ, Lufkin J, Hauben J, Johnson JR, Bodick N. Synovial and systemic pharmacokinetics (PK) of triamcinolone acetonide (TA) following intra-articular (IA) injection of an extended-release microsphere-based.

For determining said local and systemic concentration of colchicine, the man skilled in the art may use any known method. For example, a LC-MS analysis may be used.

Illustration of said particular control of the release of colchicine as achieved in the framework of the present invention may also be illustrated *via in vitro* dissolution profiles as performed in examples 1 to 3.

According to one embodiment, the dissolution medium that may be used for performing such dissolution test may be a Phosphate Buffer 50 mM (pH 7.4).

According to one embodiment, the rotation speed may range from 50 to 200 rpm, in particular from 60 to 100 rpm. This agitation may be obtained thanks to shaking incubator, in particular with horizontal motion.

According to one embodiment, the dissolution of colchicine is measured for example by UV analysis using a UV spectrophotometer, for example at 350 nm, at different intervals, for example at 24 hours, one month, three months and six months.

By way of example, a dissolution test for colchicine microparticles may be performed according to the herein after detailed method:
Aliquots of 20 mg microparticles are suspended in 50 mL of Phosphate Buffer (50 mM, pH 7.4) containing 0.02% sodium azide and maintained at 37°C in a horizontal shaker at 80 rpm. At different intervals, samples of 3 mL of the media are withdrawn and replaced with fresh medium. The removed sample is centrifuged at 4000 rpm for 3 minutes. The supernatant is analyzed by UV at 350 nm.

As above mentioned, the controlled release form is administered locally to treat pain and inflammation in the joints of patients subject to a joint disease, such as osteoarthritis and any other type of chondropathy and/or a pain-associated joint disease, in particular osteoarthritis, including osteoarthritis patients with inflammatory component, inflammatory pain or joint effusion. Local administration of the formulation may occur by injection into the intra-articular space or peri-articular space at or near the site of a patient's pain, including the metatarsophalangeal joints, the metacarpophalangeal joints, the knee joint, the shoulder joint, the wrist joint, the elbow joint, the ankle joint, the hip joint, a vertebral joint and the midfoot joints. The midfoot is the anterior part of the foot that sits between the hindfoot and forefoot. It is composed of the cuboid, navicular and three cuneiform bones. The mid-tarsal and the tarsometatarsal joints join the midfoot to the hindfoot and the forefoot respectively. The midfoot is a complex and composite area where observed oedema inflammation is generally diffuse.

In one embodiment, the present invention is dedicated to the treatment of pain-associated with osteoarthritis in joints of patients subject to osteoarthritis.

In another embodiment, the present invention is dedicated to the treatment of osteoarthritis patients with inflammatory component, inflammatory pain or joint effusion.

The administration may be performed by a single injection provided that the time required to release 80% by weight of the colchicine in the microparticles is greater than one month, in particular lies between one month and six months, the colchicine being present in a concentration ranging from 0.003 to 27.5 mg per ml of the sterile and injectable dosage form, in particular a suspension, and the pharmaceutical composition having for example a volume ranging from 0.1 ml to 5 ml. In other words, the single injection provides locally a colchicine total weight ranging between 0.0003 to 137.5 mg, i.e. amount of colchicine that is released within the total time period of the release after injection.

In one embodiment, the injection is performed a plurality of times per year depending on the release time of the composition (two times, three times, four times or more). In this embodiment, each subsequent time the injection is performed after a suitable period has lapsed since the preceding time the injection was performed. This suitable time can be, for example, two weeks or one month.

According to a particular embodiment, the colchicine is present in a concentration ranging from 0.003 to 27.5 mg per ml of the sterile and injectable dosage form, in particular a suspension, in particular from 0.003 to 13.75 mg per ml.

In a particular embodiment, the same pharmaceutical composition under the form of a suspension may be used for intra-articular injection to any painful joint of a patient subject to osteoarthritis. The amount of pharmaceutical composition to be injected may however be adapted to the targeted joint. This is one of the advantages of the invention, which may with a single concentration offer the adapted amount of colchicine for each site of application by adapting the corresponding needed volume.

In one embodiment, injections may be performed simultaneously in two or more different painful joints.

For example, the volume of pharmaceutical composition, in particular under the form of a suspension, according to the present invention that may be injected to the shoulder may vary between 2 and 5 ml.

The volume of pharmaceutical composition, in particular under the form of a suspension, according to the present invention that may be injected to the metatarsophalangeal joints, for example the toes, may vary between 0.1 and 0.5 ml, typically is 0.25 ml.

The volume of pharmaceutical composition, in particular under the form of a suspension, according to the present invention that may be injected to the metacarpophalangeal joints, for example the fingers, may vary between 0.1 and 0.5 ml, typically is 0.25 ml.

The volume of pharmaceutical composition, in particular under the form of a suspension, according to the present invention that may be injected to a vertebral joint may vary between 1 and 2 ml.

The volume of pharmaceutical composition, in particular under the form of a suspension, according to the present invention that may be injected to the midfoot joints may vary between 0.1 and 1 ml.

The volume of pharmaceutical composition, in particular under the form of a suspension, according to the present invention that may be injected to the knee, may vary between 2 and 5 ml.

The volume of pharmaceutical composition, in particular under the form of a suspension, according to the present invention that may be injected to the wrist may vary between 0.5 and 1 ml.

The volume of pharmaceutical composition, in particular under the form of a suspension according to the present invention that may be injected to the elbow may vary between 2 and 5 ml.

The volume of pharmaceutical composition, in particular under the form of a suspension, according to the present invention that may be injected to the ankle may vary between 1 and 3 ml.

The volume of pharmaceutical composition, in particular under the form of a suspension, according to the present invention that may be injected to the hip may vary between 2 and 5 ml.

In one embodiment, the pharmaceutical composition, in particular under the form of a suspension, according to the present invention may be characterized by its volume, which may be adapted to the joint, and namely may range from 2 to 5 ml for the shoulder, from 1 to 2 ml for a vertebral joint, from 0.1 to 1 ml for the midfoot joints, from 0.1 to 0.5 ml for the metatarsophalangeal joints, from 0.1 to 0.5 ml for the metacarpophalangeal joints, from 2 to 5 ml for the knee joint, from 0.5 to 1 ml for the wrist joint, from 2 to 5 ml for the elbow joint, from 1 to 3 ml for the ankle joint and from 2 to 5 ml for the hip.

It should also be understood that a specific dosage and treatment regimen for any particular patient will depend upon a variety of factors, including the age, body weight, general health, sex, diet, time of administration, drug combination, and the judgment of the treating physician and the severity of the particular disease being treated.

In one embodiment, the controlled release is suitable for reaching an articular colchicine maximum concentration (Cmax) ranging from 5 nM (2ng/mL) to 5 µM (2 µg/mL).

According to a preferred embodiment, the injection of the pharmaceutical composition of the present invention allows reaching a systemic concentration of colchicine 24 hours after the intra-articular injection and within the total time period of the release after injection, in particular over 1 month, and even more particularly over at least 3 months, which does not exceed 5 ng/mL, in particular not exceeding 2 ng/ml and more particularly not exceeding 1 ng/ml, more particularly not exceeding 0.5 ng/ml, and even more particularly not exceeding 0.1 ng/ml.

In addition, the injection of the controlled release pharmaceutical composition according to the invention can be able to achieve a synovial concentration of colchicine 24 hours after the intra-articular injection of colchicine, over 1 month, and even more particularly over at least 3 months, greater than 0.5 ng/ml, in particular comprised between 0.5 and 100 ng/ml and more particularly comprised between 0.5 and 50 ng/ml.

The requested dose to be injected into the painful joint can depend on the type of painful joint, in particular depending on its size. Table 1a as follows gathers typical dosage of colchicine that may be implemented in the framework of the present invention.

According to one embodiment, the pharmaceutical composition conform to the present invention has a volume ranging from 0.1 ml to 5 ml.

The volume corresponds to the total volume of the composition suitable for the injection, i.e. in particular under the form of a suspension.

**Table 1:**

| | **Joint** | **Volume (ml)** | **Drug concentration (mg/ml)** | **Dose range (mg)** |
|---|---|---|---|---|
| **Small joints** | Metatarsophalangeal | 0.1-0.5 | 0.003 - 27.5 | 0.0003 - 13.75 |
| | | | [0.003 - 13.75]^{∗} | [0.0003 - 6.875]^{∗} |
| | Metacarpophalangeal | 0.1-0.5 | 0.003 - 27.5 | 0.0003 - 13.75 |
| | | | [0.003 - 13.75]^{∗} | [0.0003 - 6.875]^{∗} |
| | wrist | 0.5-1 | 0.003 - 27.5 | 0.0015 - 27.5 |
| | | | [0.003 - 13.75]^{∗} | [0.0015 -13.75]^{∗} |
| | midfoot | 0.1-1 | 0.003 - 27.5 | 0.0003 - 27.5 |
| | | | [0.003 - 13.75]^{∗} | [0.0003 - 13.75]^{∗} |
| **Medium/large joints** | vertebral | 1-2 | 0.003 - 27.5 | 0.003 - 55 |
| | | | [0.003 - 13.75]^{∗} | [0.003 - 27.5]^{∗} |
| | ankle | 1-3 | 0.003 - 27.5 | 0.003 - 82.5 |
| | | | [0.003 - 13.75]^{∗} | [0.003 - 41.25]^{∗} |
| | knee | 2-5 | 0.003 - 27.5 | 0.006 - 137.5 |
| | | | [0.003 - 13.75]^{∗} | [0.006 - 68.5]^{∗} |
| | hip | 2-5 | 0.003 - 27.5 | 0.006 - 137.5 |
| | | | [0.003 - 13.751^{∗} | [0.006 - 68.5]^{∗} |
| | shoulder | 2-5 | 0.003 - 27.5 | 0.006 - 137.5 |
| | | | [0.003 - 13.75]^{∗} | [0.006 - 68.5]^{∗} |
| | elbow | 2-5 | 0.003 - 27.5 | 0.006 - 137.5 |
| | | | [0.003 13.75]* | [0.006 - 68.5]* |

| | | | | |
|---|---|---|---|---|
| ^{∗}[in particular] | | | | |

In one embodiment, the pharmaceutical composition, in particular under the form of a suspension, in the present invention, which is in particular administered in one intra-articular injection, and which is dedicated to the treatment of a joint disease such as osteoarthritis and any other type of chondropathy and/or a pain-associated joint disease, in particular osteoarthritis and/or a pain-associated osteoarthritis, comprises between 0.0003 and 13.75 mg of colchicine per injection for a small joint selected from metatarsophalangeal joints or metacarpophalangeal joints.

In another embodiment, the pharmaceutical composition, in particular under the form of a suspension, in the present invention, which is in particular administered in one intra-articular injection, and which is dedicated to the treatment of a joint disease such as osteoarthritis and any other type of chondropathy and/or a pain-associated joint disease, in particular osteoarthritis and/or a pain-associated osteoarthritis, comprises between 0.0015 and 27.5 mg of colchicine per injection for a wrist joint.

In another embodiment, the pharmaceutical composition, in particular under the form of a suspension, in the present invention, which is in particular administered in one intra-articular injection, and which is dedicated to the treatment of a joint disease such as osteoarthritis and any other type of chondropathy and/or a pain-associated joint disease, in particular osteoarthritis and/or a pain-associated osteoarthritis, comprises between 0.0003 and 27.5 mg of colchicine per injection for midfoot joints.

In another embodiment, the pharmaceutical composition, in particular under the form of a suspension, in the present invention, which is in particular administered in one intra-articular injection, and which is dedicated to the treatment of a joint disease such as osteoarthritis and any other type of chondropathy and/or a pain-associated joint disease, in particular osteoarthritis and/or a pain-associated osteoarthritis, comprises between 0.003 and 82.5 mg of colchicine per injection, for an ankle joint or 0.003 and 55 mg of colchicine per injection in vertebral joint.

In another embodiment, the pharmaceutical composition, in particular under the form of a suspension, in the present invention, which is in particular administered in one intra-articular injection, and which is dedicated to the treatment of a joint disease such as osteoarthritis and any other type of chondropathy and/or a pain-associated joint disease, in particular osteoarthritis and/or a pain-associated osteoarthritis, comprises between 0.006 and 137.5 mg of colchicine per injection for a joint selected from a knee joint, a hip joint, a shoulder joint and an elbow joint.

According to one embodiment, the pharmaceutical composition, in particular under the form of a suspension, in the present invention, which is in particular administered in one intra-articular injection, and which is dedicated to the treatment of a joint disease such as osteoarthritis and any other type of chondropathy and/or a pain-associated joint disease, in particular osteoarthritis and/or a pain-associated osteoarthritis, comprises between 0.003 to 27.5 mg/ml, in particular 0.003 to 13.75 mg/ml of colchicine.

In one embodiment, the pharmaceutical composition may be suited for maintaining a therapeutically acceptable concentration of colchicine in the painful joint during a period greater than one month and inferior to six months, a period greater than two months and inferior to six months, a period greater than three months and inferior to six months.

Thus, herein is provided a pharmaceutical composition under the form of a sterile and injectable dosage form for use according to the present invention, wherein the time required to release 80% by weight of the colchicine lies between one to six months, two to six months or three to six months.

In one embodiment, the pharmaceutical composition under the form of a sterile and injectable dosage form for use according to the invention, is characterized by the fact that the time required to release 80% by weight of the colchicine in the microparticles is superior to 1 month, for example 1.5 month, and may reach 6 months, is superior to 2 months, for example 2.5 months, and may reach 6 months, is superior to 3 months, for example 3.5 months, and may reach 6 months, and for example is superior to 4 months, for example 4.5 months or 5 months, and may reach 6 months.

According to one embodiment, the pharmaceutical composition may be suited for providing an initial articular colchicine concentration.

The pharmaceutical composition may or may not exhibit a burst release. In the framework of the present invention a "burst release" means that an initial bolus of drug is released before the release rate reaches a stable profile, immediately upon placement in the release medium, i.e. in the present invention immediately after the injection into the articulation.

Thus, when a burst release occurs, upon administration, the pharmaceutical composition according to the present invention may provide an initial release of colchicine at the site of administration, for example, in the intra-articular space and/or peri-articular space. Once the initial release of colchicine has subsided, the controlled release of the colchicine microparticle formulation continues to provide therapeutic (e.g., intra-articular and/or peri-articular) concentrations of colchicine to combat inflammation and/or pain for an additional period of therapy following administration.

In a particular embodiment, the pharmaceutical composition for use according to the present invention does not exhibit an important burst release. According to said embodiment, less than 25% by weight of colchicine is released at 24 hours after the administration.

According to another aspect, the invention relates to a method for the treatment of a joint disease, such as osteoarthritis and any other type of chondropathy and/or a pain-associated joint disease, in particular osteoarthritis and/or a pain-associated osteoarthritis comprising at least the administering by injection to a painful joint to a patient in need thereof of a pharmaceutical composition under the form of a sterile and injectable dosage form comprising a controlled release dosage form comprising an effective amount of colchicine, suitable for intra-articular injection into a joint, wherein the time required to release 80% by weight of the colchicine in the microparticles is greater than one month, in particular is between one and six months, wherein the colchicine is present in a concentration ranging from 0.003 to 27.5 mg per ml of the sterile and injectable dosage form, in particular a suspension, in particular from 0.003 to 13.75 mg per ml.

Still according to another aspect, the invention relates to a method for the treatment of a joint disease, such as osteoarthritis and any other type of chondropathy and/or a pain-associated joint disease, in particular osteoarthritis and/or a pain-associated osteoarthritis comprising at least the administration by injection into the joints of a patient in need thereof of at least an efficient amount of colchicine for a controlled release, in particular wherein the time required to release 80% by weight of the colchicine is greater than one month, in particular is between one and six months.

Herein is further provided a method for the treatment of a joint disease, such as osteoarthritis and any other type of chondropathy and/or a pain-associated joint disease, in particular osteoarthritis and/or a pain-associated osteoarthritis comprising at least the steps of:
- preparing and/or providing a pharmaceutical composition under the form of a sterile and injectable suspension by mixing a formulation under the form of a powder as described herein after, with an aqueous injection vehicle, wherein the pharmaceutical composition optionally comprises an excipient selected from a group consisting of a tonicity-enhancing agent, a wetting-agent, a viscosity-enhancing agent, a density-enhancing agent or a mixture thereof, and wherein said excipient may be present in the aqueous injection vehicle or in the powder, and
- injecting said pharmaceutical composition into a painful joint of a patient in need thereof, wherein the volume to be injected is adapted to the injection site.

The present invention may be particularly suitable for treating patients having renal and/or hepatic impairment of patients being simultaneously treated with drugs. Indeed, for patients having renal and/or hepatic impairment, adjustments of the recommended oral dosage of colchicine are required for adaptation of the administration scheme. Moreover, some drugs have been identified in the past with oral colchicine treatments as generating undesirable side effects and even fatal drug interactions, drastically modifying the safety profile and necessitating oral colchicine dosage adjustments which may for example require a reduction by two to three times of the oral colchicine dosage.

Thus, in one embodiment, the pharmaceutical composition conform to the present invention may be administered to patients having renal and/or hepatic impairment. In particular, colchicine plasmatic level is deemed to rise to toxicity threshold when intrinsic factor renal or hepatic impairment achieve Severe grade.

Patients having renal impairment may be identified and classified by techniques known to the physicians such as *via* the Creatinine Clearance method or *via* the Glomerular Filtration Rate (GFR) method. Said methods are in particular detailed in Worboys PD, Wong SL, Barriere SL. Pharmacokinetics of intravenous telavancin in healthy subjects with varying degrees of renal impairment. Eur J Clin Pharmacol. 2015 Jun;71(6):707-714. The use and method according to the present invention may for example be implemented to treat patients having all grades of renal impairment such as grades 1 to 5 as set by the GFR method. The use and method according to the present invention may be more particularly suited for patients having high grades of renal impairment, such as grades 3 to 5 as set by the GFR method.

Patients having hepatic impairment may be identified and classified by techniques known to the physicians such as *via* the Score of Child-pugh method or *via* the MELD score. Said methods are in particular detailed in Tsoris A, Marlar CA. Use Of The Child Pugh Score In Liver Disease. 2021 Mar 22. In: StatPearls [Internet]. Treasure Island (FL): StatPearls Publishing; 2022 Jan-. PMID: 31194448 or on internet under the following links: https://liverfellow.org/post/meld-score-part1 and https://liverfellow.org/post/meld-score-part2. The use and method according to the present invention may for example be implemented to treat patients having all scores, rates and/or classification, for example mild, moderate of severe hepatic impairment as set by the Child-Pugh method. The use and method according to the present invention may be more particularly suited for patients having high scores, rates and/or classification of severe hepatic impairment as set by the Child-Pugh method such as moderate and severe hepatic impairment.

Thus, due to the colchicine systemic plasmatic level being maintained under the threshold leading to toxicity in the framework of the present invention, no colchicine dosage adjustment is required even for treating patients with intrinsic factor impacts, such as renal and/or hepatic impairment that currently require colchicine dosage adjustments when orally administered.

Thus, in one embodiment, the pharmaceutical composition for use according to the present invention may further be characterized by the fact that the patient to which said pharmaceutical composition is administered have renal and/or hepatic impairment.

In one other embodiment, further provided a method for the treatment of a joint disease, such as osteoarthritis and any other type of chondropathy and/or a pain-associated joint disease, in particular osteoarthritis and/or a pain-associated osteoarthritis to a patient having renal and/or hepatic impairment, in particular wherein the dosage of colchicine is not reduced in response to such impairments.

In one embodiment, the pharmaceutical composition conform to the present invention may be co-administered with a wide range of drugs as detailed herein after, in particular showing improved safety profiles in comparison to the classical treatment with oral colchicine when co-administered with such drugs.

As an example of drug interaction that the present invention allows to avoid are the interactions with drugs that have been clearly identified in the framework of treatments with oral colchicine and that are well known to the physicians. In a situation of potential drug interaction, the classical way to proceed consists in adjusting, namely lowering the dosage of oral colchicine, depending on the severity of the interaction, according to well-known recommendations.

Such drug drug interactions (DDI) may be explained by the fact that some drugs inhibit P-gp and/or CYP3A4, generating peaks of excessive plasmatic concentration of colchicine, and in particular peaks above 6 ng/mL of colchicine, that must be avoided.

One advantage of the present invention relies in the fact that excessive plasmatic concentration is avoided and allows co-administration of drugs without adjusting the colchicine dosage that must be done when orally administered.

Thus, in one embodiment, the pharmaceutical composition for use according to the present invention may further be characterized by the fact that the patient to which said pharmaceutical composition is administered may simultaneously, separately or sequentially be treated by the following active ingredients: atazanavir, clarithromycin, darunavir, ritonavir, indinavir, itraconazole, ketoconazole, lopinavir, , nefazodone, nelfinavir, , saquinavir, telithromycin, tipranavir, amprenavir, aprepitant, diltiazem, erythromycin, fluconazole, fosamprenavir, verapamil, cyclosporine and ranolazine.

Herein is thus provided the pharmaceutical composition for use as defined above wherein it is for use in the treatment of a joint disease, such as osteoarthritis and any other type of chondropathy and/or a pain-associated joint disease, in particular osteoarthritis and/or a pain-associated osteoarthritis in a patient being simultaneously, separately or sequentially and non-exclusively treated by at least one of the active ingredient selected from: atazanavir, clarithromycin, darunavir, ritonavir, indinavir, itraconazole, ketoconazole, lopinavir, nefazodone, nelfinavir, saquinavir, telithromycin, tipranavir, amprenavir, aprepitant, diltiazem, erythromycin, fluconazole, fosamprenavir, verapamil, cyclosporine, ranolazine, macrolides and statins.

Herein is thus further provided a method for the treatment of a joint disease, such as osteoarthritis and any other type of chondropathy and/or a pain-associated joint disease, in particular osteoarthritis and/or a pain-associated osteoarthritis to a patient being simultaneously, separately or sequentially treated by at least one active ingredient selected from: atazanavir, clarithromycin, darunavir, indinavir, itraconazole, ketoconazole, lopinavir, ritonavir, nefazodone, nelfinavir, saquinavir, telithromycin, tipranavir, amprenavir, aprepitant, diltiazem, erythromycin, fluconazole, fosamprenavir, verapamil, cyclosporine and ranolazine, in particular wherein the dosage of colchicine is not reduced in response to the concomitant administration of such drug.

### CONTROLLED RELEASE DOSAGE FORM COMPRISING COLCHICINE

According to one embodiment, the controlled release dosage form comprising colchicine is under the form of an in-situ forming depot or microparticles, in particular microparticles comprising a polymeric matrix or multivesicular liposomes.

In one embodiment, the controlled release dosage form comprising colchicine is under the form of an in-situ forming depot. An in-situ forming depot, such as pH-induced, thermally-induced or solvent exchange-induced gelling systems comprising colchicine may be prepared according to techniques known to the man skilled in the art, as in particular described in L. Rahnfeld et al. Injectable Lipid-Based Depot Formulations: Where Do We Stand?. Pharmaceutics. 2020, 12(6):567, in S. Kempe et al. In-situ forming implants-an attractive formulation principle for parenteral depot formulations Journal of Controlled Release 2012, 161:668.

In one embodiment, the controlled release dosage form comprising colchicine is under the form of microparticles. Said embodiments are described in detail herein after.

### CONTROLLED RELEASE MICROPARTICLES COMPRISING COLCHICINE

The controlled release microparticles comprising colchicine may take the form of various microparticles, such as (i) microparticles comprising a polymeric matrix or (ii) multivesicular liposomes.

According to one embodiment, the microparticles have a mean particle size determined by laser light diffraction measurement method equal or greater than 10 µm.

According to another embodiment, the microparticles have a mean particle size determined by laser light diffraction measurement method equal or greater than 10 µm, in particular a mean particle size lower than 100 µm, more particularly lower than 80 µm, and even more particularly lower than 50 µm, for example between 10 and 50 µm or between 10 and 40 µm.

The particle size may be measured in the framework of the present invention by laser light diffraction measurement method.
The equipment may typically be a Malvern Mastersizer MS3000 laser diffraction particle size analyzer. A preparation of 0.1 to 1 mg of microparticles per ml of deionized water may be introduced in the apparatus. The measurement may be run three times. Each run may last for 3 seconds, and the suspension may be homogenized before each run.

It is understood that these ranges refer to the mean size of all microparticles in a given population. The size of any given individual microparticle could be within a standard deviation above or below the mean size.

Within the context of the present invention, a "microparticle" means a particle of any shape and made of any material, in particular suitable for the injection into the human body within a pharmaceutical composition, and in particular into the articulations or joints, having a mean particle size determined by laser light diffraction measurement method greater than 10 µm and less than 100 µm.

The microparticles suitable for the pharmaceutical composition used according to the present invention may be selected form various types of microparticles such as microspheres, microparticle matrices, microsphere matrices, microcapsules, rods, wafers, pills, fibers and pellets.

### (i) MICROPARTICLES COMPRISING A POLYMERIC MATRIX

The polymeric matrix may be chosen from various polymers suitable for obtaining the controlled release microparticles. Such polymeric matrix is nontoxic to the human body.

According to one embodiment, the polymer forming the polymeric matrix is biodegradable.

Within the context of the present invention, a "biodegradable" material, refers to a material which degrades enzymatically or hydrolytically and for which there is proof that the degradation products are integrated into biomass and/or eliminated from the organism by metabolism or renal filtration.

The nontoxic and biodegradable polymer may be natural or synthetic.

According to one embodiment, the polymeric matrix of the microparticles used in the pharmaceutical composition used according to the present invention comprises at least a poly(lactic-co-glycolic acid) copolymer or PLGA.

According to said embodiment, the polymeric matrix may comprise PLGA in an amount of greater than 70% by weight, with respect to the total weight of the polymeric matrix, in particular greater than 80% by weight, and even more particularly greater than 90% by weight.

Suitable polymers are not limited to those commercially available and known as RESOMER (Evonik Industries AG, Germany), LACTEL (Durect, USA), PURASORB (Corbion NV, The Netherlands), Viatel (Ashland, USA), EXPANSORB (Seqens, FRANCE).

According to another embodiment, the polymeric matrix of the microparticles used in the pharmaceutical composition used according to the present invention comprises at least a poly(caprolactone) (PCL).

According to said embodiment, the polymeric matrix may comprise PCL in an amount of greater than 70% by weight, with respect to the total weight of the polymeric matrix, in particular greater than 80% by weight, and even more particularly greater than 90% by weight.

According to another embodiment, the polymeric matrix of the microparticles used in the pharmaceutical composition according to the present invention comprises a mixture of at least a PCL and at least a PLGA.

In a particular embodiment, the pharmaceutical composition according to the present invention is characterized by the microparticles, which may be a mixture of microparticles of different nature, which are microparticles comprising colchicine and a polymeric matrix, wherein the polymeric matrix comprises at least a poly(lactic-co-glycolic acid) copolymer, at least poly(caprolactone) or at least a mixture of poly(lactic-co-glycolic acid) copolymer and poly(caprolactone).

By "which may be a mixture of microparticles of different nature" is meant that the pharmaceutical composition may comprise various types of microparticles, for example, 2, 3 or 4 types of microparticles. By "type of microparticles" is meant microparticles having polymer matrices of different nature, in particular by way of exhibiting particular proportions of monomers, molecular weight and/or inherent viscosity. By way of example, one type of microparticle may have a polymer matrix comprising at least, or even consisting in a poly(lactic-co-glycolic acid) copolymer and another type of microparticles may have a polymer matrix comprising at least, or even consisting in, poly(caprolactone) or one type of microparticle may have a polymer matrix comprising at least, or even consisting in a poly(lactic-co-glycolic acid) copolymer exhibiting a particular proportion of monomers, molecular weight and/or inherent viscosity and another type of microparticles may have a polymer matrix comprising at least, or even consisting in a poly(lactic-co-glycolic acid) copolymer exhibiting a different particular proportions of monomers, molecular weight and/or inherent viscosity. All types of mixtures are thus encompassed within the scope of the present invention, in particular for the benefit of obtaining a release profile suitable for the treatment of a joint disease, such as osteoarthritis and any other type of chondropathy and/or a pain-associated joint disease, in particular osteoarthritis and/or a pain-associated osteoarthritis.

As far as poly(lactic-co-glycolic acid) copolymer or PLGA is concerned, depending on the molar ratio of lactide to glycolide used for the polymerization, different forms of PLGA can be obtained. Thus, a PLGA as used in the present invention may be characterized by its lactic:glycolic ratio. In the framework of the present invention, the "lactic:glycolic ratio" means the molar ratio between the used monomers lactic acid and glycolic acid. By way of example, "PLGA 75:25" or a "PLGA having a lactic:glycolic molar ratio of 75:25" means a PLGA whose composition is 75% lactic acid and 25% glycolic acid.

In one embodiment, the poly(lactic-co-glycolic acid) copolymer exhibits an inherent viscosity determined by an Ubbelohde capillary viscometer of 0.10 to 1.7 dl/g, in particular of 0.10 to 1.4 dl/g and even more particularly from 0.10 to 0.9 dl/g.

In the framework of the present invention "inherent viscosity" (IV) is a measure of the increased resistance to flow of a polymer solution compared to the pure solvent. This property is related to molar mass: longer polymer chains lead to higher values of inherent viscosity.

The inherent viscosity may typically be measured according to the following method of measurement: The inherent viscosity of polymer samples may be determined with a Ubbelohde capillary viscometer. The polymer is dissolved in chloroform. The flow time of solvent and polymer solution is measured analog USP <911> and the inherent viscosity is calculated.

In one embodiment, the polymeric matrix comprises at least one poly(lactic-co-glycolic acid) copolymer, at least one poly(caprolactone), or a mixture of at least one poly(lactic-co-glycolic acid) copolymer and at least one poly(caprolactone) in an amount of greater than 70% by weight, with respect to the total weight of the polymeric matrix, in particular greater than 80% by weight, and even more particularly greater than 90% by weight, wherein the poly(lactic-co-glycolic acid) copolymer has in particular a lactic:glycolic molar ratio ranging between 50:50 and 90:10, in particular ranging between 60:40 and 90:10, more particularly ranging between 65:35 and 85:15, for example 75:25, and wherein the poly(lactic-co-glycolic acid) copolymer in particular exhibits an inherent viscosity determined by an Ubbelohde capillary viscometer of 0.1 to 1.7 dl/g, in particular of 0.1 to 1.4 dl/g and even more particularly from 0.1 to 0.9 dl/g.

According to said embodiment, the polymeric matrix may comprise a mixture of PCL and PLGA in an amount of greater than 70% by weight, with respect to the total weight of the polymeric matrix, in particular greater than 80% by weight, and even more particularly greater than 90% by weight.

The polymeric matrix may comprise one or more additional polymer(s), copolymer(s) or mixture thereof. Said additional polymer(s), copolymer(s) or mixture thereof may be present in the polymer matrix in an amount ranging from 0 to 30% by weight, in particular from 0 to 20% by weight, and more particularly from 0 to 10% by weight, with respect to the total weight of the polymeric matrix.

Non-limiting examples of suitable additional polymers or copolymers include poly(lactide) or PLA distinct from poly(lactic-co-glycolic acid) copolymer, poly(glycolide) or PGA distinct from poly(lactic-co-glycolic acid) copolymer, poly(lactide-co-caprolactone), poly(ethylene glycol), poly(ethylene oxide) or PEO, PLGA-b-PEO-b-PLGA, PLGA-b-PEO, polyhydroxyalkanoates, poly(hydroxybutyrate), poly(trimethylene carbonate), poly(dioxanone), poly(valerolactone), poly(alpha- hydroxy acids), poly(lactones), poly(amino acids), polyanhydrides, poly(orthoester), poly(acetals), polyurethanes, polythioesters, polyphosphoesters, poly(ester-co-amide), poly(vinyl alcohol) or PVA, PVA-g-PLGA, poly (ether ester) multiblock copolymers, polyvinyl pyrrolidone, poly(methacrylate), PEO-PPO-PEO (pluronics), gelatin, heparin, chondroitin sulfate; polysaccharides such as alginates, starch, chitosan and dextrans and any combinations thereof.

In one embodiment, the controlled release microparticles according to the present invention are PLGA microspheres. In other words, in said embodiment, the polymeric matrix does not comprise any additional polymer or copolymer. In such an embodiment, the controlled release microparticles may comprise different types of PLGA microspheres, that is to say may comprise PLGA microspheres manufactured with poly(lactic-co-glycolic acid) copolymer exhibiting different lactic:glycolic molar ratio and/or inherent viscosity.

According to another embodiment, a microparticle matrix can comprise, and even may consist in, a blend of two, three or more PLGA copolymers, in particular two PLGA copolymers. In the present description "the polymeric matrix comprising at least one poly(lactic-co-glycolic acid) copolymer" precisely means that the poly(lactic-co-glycolic acid) copolymer may be such a blend.

The microparticle may further comprise pharmaceutically acceptable excipients for modulating the drug release profile such as medium chain triglycerides, poly(oxyethylene) sorbitan fatty acid esters (e.g. polysorbate 20, polysorbate 80), sorbitan fatty acid esters, cyclodextrins, lecithin, mannitol, sucrose, inorganic salts and mixtures thereof.

The polymeric matrix of the microparticles may comprise one or more excipient(s). Said excipient(s) may be present in the polymer matrix in an amount not exceeding 15% by weight, in particular not exceeding 10% by weight, and more particularly not exceeding 5% by weight, with respect to the total weight of the polymeric matrix.

In one embodiment, the percentage weight ratio between the colchicine and the total weight of the microparticles or colchicine loading content in the pharmaceutical composition ranges from 0.01 to 40% by weight.

### Manufacturing process for the obtention of the microparticles comprising a polymer matrix

Any process suitable to produce polymeric microparticles with a mean particle size determined by laser light diffraction measurement method ranging from 10 µm to 100 µm is considered appropriate in the framework of the present invention.

Among such manufacturing process emulsification-based processes, such as high-pressure homogenization, for example using rotor-stator homogenizer in a batch or continuous mode, or membrane emulsification, followed by organic solvent removal by extraction/evaporation may be cited.

As far as the general principal of such methods is concerned, an emulsion may be prepared and processed over a membrane with pores with a determined size. The obtained microspheres can then be collected after extraction and evaporation of the organic solvent, washed and freeze-dried.

According to one particular embodiment, the microparticles may be manufactured from an O/W direct emulsion or from a W/O/W double emulsion technique.

Schoubben, A., Ricci, M. & Giovagnoli, S. Meeting the unmet: from traditional to cutting-edge techniques for poly lactide and poly lactide-co-glycolide microparticle manufacturing. J. Pharm. Investig. 49, 381-404 (2019) reviews various methods to make PLGA microparticles that can be used in the framework of the present invention.

According to one particular embodiment, the microparticles may be manufactured from a Solid-in-Oil-in-Water (S/O/W) double emulsion technique.

Giovagnoli, S., et al.; Physicochemical characterization and release mechanism of a novel prednisone biodegradable microsphere formulation, J Pharm Sci. 97:303-317, (2008) describes an example of PLGA microparticles prepared *via* S/O/W emulsion technique. Other manufacturing approaches suitable for obtaining the microparticles according to the present invention are atomization by spinning disk, atomization by spraydrying, a fluidized bed coating, or combinations thereof.

Alternatively, the microparticles may be manufactured using drop-on-demand, drop-by-drop and jet break-up processes such as inkjet printing or microfluidics.

Alternatively, the microparticles may be manufactured using supercritical fluid technologies.

Alternatively, the microparticles may be manufactured using microfabrication methods, such as template and mold-based techniques, such as soft lithography.

All these manufacturing processes are well known by the man skilled in the art.

The hereabove cited manufacturing processes are well known to the man skilled in the art.

### (ii) MULTI VESICULAR LIPOSOMES

Multivesicular liposomes (MVL) are spherical particles with a mean diameter of 10-30 µm and composed of non-concentric multiple lipid bilayers arranged in a honeycomblike structure. These lipid layers enclose numerous water-filled aqueous compartments that can be used to encapsulate a water-soluble drug such as colchicine.

MVLs are classically composed of at least one amphiphilic lipid and one neutral lipid. The amphiphilic lipid is selected from phospholipids for instance phosphatidylcholine or phosphatidylglycerol. The neutral lipid is selected from triglycerides having monounsaturated fatty acid ester moieties containing 14-18 carbons in the acyl chain (e.g. triolein, tripalmitolein), saturated fatty acid ester moieties containing 6 to 8 carbons in the acyl chain (e.g. tricaproin, tricaprylin) and mixtures thereof. Cholesterol can also be used in the composition.

MVLs are obtained by using a water-in-oil-in-water (W/O/W) double emulsification process. In a first step, a water-in-oil (W/O) emulsion is prepared by mixing phospholipids, triolein, tricaprylin and cholesterol solubilized in volatile and waterimmiscible organic solvent with an aqueous solution containing the solubilized drug to be encapsulated. This first emulsion is then emulsified by mixing with a second aqueous solution to produce the water-in-oil-in-water (W/O/W) emulsion. The energy needed to form the first and second emulsion can be provided either mechanically, by sonication or by combinations thereof. MVLs are finally obtained by removal of the volatile organic solvent from the double emulsion using gas stripping or flushing. Finally, removal of unencapsulated material, concentration of the MVLs and buffer exchange is performed using either diafiltration or cross-flow filtration system.

In one embodiment, the neutral lipids used to manufacture the MVL encapsulating colchicine comprises a mixture of triolein: tricaprylin with ratios ranging from 50:50 to 0:100.

### PHARMACEUTICAL COMPOSITION AND KITS

As mentioned above, herein are provided pharmaceutical compositions in various forms, i.e. under the form of a solution, a suspension, a solid implant, a semi-solid implant, a powder and an in-situ forming depot. Further pharmaceutical compositions are provided under the form of a powder or under the form of a kit. When under the form of a powder, the pharmaceutical composition is mainly aimed for storage whereas the solution, suspension, solid implant, semi-solid implant, powder or in-situ depot, in particular the suspension, is the ready to use composition, ready for injection and the kit allows to store separately (i) an aqueous injection vehicle and (ii) a controlled release dosage form comprising colchicine, in particular under the form of a powder for forming a sterile and injectable dosage form, in particular a suspension, suitable for the injection.

In one embodiment, the pharmaceutical composition is further characterized in that it is in particular under the form of a sterile and injectable suspension, optionally comprising an excipient selected from a group consisting of a tonicity-enhancing agent, a wetting-agent, a viscosity-enhancing agent, a density-enhancing agent or a mixture thereof, and wherein it is obtained by mixing controlled release dosage form comprising colchicine, in particular microparticles comprising colchicine, more particularly as defined above with an aqueous injection vehicle.

Various embodiments of said alternative forms are detailed herein after.

Solution, solid implant, semi-solid implant, powder and in-situ forming depot may be prepared according to methods known to the man skilled in the art.

According to a particular embodiment, the sterile and injectable dosage form is under the form of a suspension that may be obtained from a powder, as detailed herein after.

### Powder and suspension

In one embodiment is provided a formulation under the form of a powder comprising a controlled release dosage form comprising colchicine, in particular under the form of microparticles comprising a polymeric matrix, more particularly as defined above, wherein the colchicine loading content in the controlled release dosage form ranges from 0.01 to 40% by weight.

In one embodiment said formulation under the form of a powder comprises a controlled release dosage form comprising colchicine, wherein said controlled release dosage form comprising colchicine is under the form of microparticles, in particular microparticles comprising a polymeric matrix, more particularly having a mean particle size determined by laser light diffraction measurement method equal or greater than 10 µm, and even more particularly as defined above.

The pharmaceutical composition used in the framework of the present invention may take the form of a sterile and injectable composition, in particular a suspension composition, comprising an effective amount of colchicine.

By "sterile", in the sense of the present invention, is meant an environment capable of guaranteeing to the considered compounds in a composition according to the invention safety requirements for the administration routes such as above-mentioned, notably into or through a joint. Indeed, for obvious reasons, it is essential that a composition according to the invention be devoid of any contaminant body capable of initiating an undesirable side reaction at the host site.

The pharmaceutical composition used in the framework of the present invention may be prepared with the formulation under the form of a powder comprising microparticles as described above. According to one embodiment, said pharmaceutical composition is a sterile and injectable composition for controlled release of colchicine suitable for an intra-articular injection.

By its injectable character, a composition according to the invention necessarily comprises a physiologically acceptable medium, also called "aqueous injection vehicle".

A "physiologically acceptable medium" means a medium devoid of toxicity and compatible with the injection and/or the application of the composition such as considered in the present invention.

In another embodiment is provided a pharmaceutical composition under the form of a sterile and injectable dosage form suitable for an intra-articular injection, obtained by mixing a formulation under the form of a powder according to the invention, with an aqueous injection vehicle, wherein the pharmaceutical composition optionally comprises an excipient selected from a group consisting of a tonicity-enhancing agent, a wetting-agent, a viscosity-enhancing agent, a density-enhancing agent or a mixture thereof, and wherein said excipient may be present in the aqueous injection vehicle or in the powder.

In another embodiment is provided a pharmaceutical composition under the form of a sterile and injectable dosage form suitable for an intra-articular injection, comprising a controlled release dosage form comprising colchicine, under the form of microparticles having a mean particle size determined by laser light diffraction measurement method equal or greater than 10 µm comprising a polymeric matrix, wherein the polymeric matrix comprises at least one poly(lactic-co-glycolic acid) copolymer, at least one poly(caprolactone), or a mixture of at least one poly(lactic-co-glycolic acid) copolymer and at least one poly(caprolactone) in an amount of greater than 70% by weight, with respect to the total weight of the polymeric matrix, in particular greater than 80% by weight, and even more particularly greater than 90% by weight, wherein the poly(lactic-co-glycolic acid) copolymer has in particular a lactic:glycolic molar ratio ranging between 50:50 and 90:10, in particular ranging between 60:40 and 90:10, more particularly ranging between 65:35 and 85:15, for example 75:25, and wherein the poly(lactic-co-glycolic acid) copolymer in particular exhibits an inherent viscosity determined by an Ubbelohde capillary viscometer of 0.1 to 1.7 dl/g, in particular of 0.1 to 1.4 dl/g and even more particularly from 0.1 to 0.9 dl/g.

In another embodiment is provided said pharmaceutical composition wherein the colchicine loading content in the controlled release dosage form ranges from 0.01 to 40% by weight.

In another embodiment, said pharmaceutical composition is selected from a solution, a suspension, a solid implant, a semi-solid implant, a powder and an in-situ depot, in particular a suspension, and is characterized by the concentration ranging from 0.003 to 27.5 mg per ml of the sterile and injectable dosage form, in particular a suspension, in particular from 0.003 to 13.75 mg per ml.

The composition may comprise a solvent or a mixture of physiologically acceptable solvents.

The composition may comprise a physiologically acceptable aqueous medium.

As an aqueous medium suitable for the invention, may be for example mentioned water.

As isotonic agents suitable for the preparation of a composition according to the invention, it may be mentioned sugars and sodium chloride.

The aqueous injection vehicle may in particular comprise a tonicity-enhancing agent, a wetting-agent, a viscosity-enhancing agent or a mixture thereof.

Among the tonicity-enhancing agent, the following may be cited: dextrose, mannitol, sorbitol, sucrose, glycerin, sodium chloride, potassium chloride, cyclodextrin and maltodextrin.

Among the wetting agents, the following may be cited: poly(oxyethylene) sorbitan fatty acid esters, such as commercially available under the trade name TWEEN, sorbitan fatty acid esters, such as commercially available under the trade name SPAN, poloxamer and lecithins.

Among the viscosity-enhancing agents, the following may be cited: sodium carboxymethyl cellulose (CMC), a glycosaminoglycan such as hyaluronic acid, dextran, collagen, poly(vinylpyrrolidone), poly(ethyleneglycol), gelatin, hydroxyethyl cellulose (HEC), methyl cellulose (MC), alginate, gum acacia, starch.

According to a particular embodiment, said pharmaceutical composition has a viscosity of from 5 to 1000 mPa.s, in particular from 5 to 500 mPa.s, in particular from 5 to 100 mPa.s and more particularly from 5 to 50 mPa.s at a shear rate of 10 s⁻¹.

### Kit

Herein is further provided a kit or article of manufacture comprising, in separate compartments (i) an aqueous injection vehicle and (ii) a controlled release dosage form comprising colchicine as defined above or powder as defined above, wherein the kit or article of manufacture optionally comprises an excipient selected from a group consisting of a tonicity-enhancing agent, a wetting-agent, a viscosity-enhancing agent, a density-enhancing agent or a mixture thereof, for preparing a pharmaceutical composition suitable for an intra-articular injection.

Herein is further provided a kit or article of manufacture comprising, in separate compartments (i) an aqueous injection vehicle and (ii) a pharmaceutical composition under the form of a powder as defined above, wherein the pharmaceutical composition optionally comprises an excipient selected from a group consisting of a tonicity-enhancing agent, a wetting-agent, a viscosity-enhancing agent, a density-enhancing agent or a mixture thereof may be present in the aqueous injection vehicle or in the powder, for preparing a pharmaceutical composition suitable for an intra-articular injection.

In one embodiment, the kit may be under the form of two separated vials.

In another embodiment, the kit or article of manufacture may be under the form of a vial and a prefilled syringe or of a medical device or is under the form of two separated vials. In said embodiment, the two compartments or dual chamber may allow the mixture of the aqueous injection vehicle and the powder as described herein after by all means known to the man skilled in the art. Still in said embodiment, said means may take the form of a pierceable membrane or destructible diaphragm, for example through a pressure the user may exert.

In these embodiments, the kit or article of manufacture may additionally contain a label instructing the user to introduce the obtained pharmaceutical composition into a subject's joint.

For the comfort of the patient and for safety reasons as explained above, it is advantageous to look for a pharmaceutical composition, suitable for intra-articular injection into a joint, requiring only one injection able to relieve the patient thanks to this injection without needing multiple injections.

### Additional active ingredients

Additional therapeutic agents, which are normally administered to treat a joint disease such as osteoarthritis and any other type of chondropathy and/or a pain-associated joint disease, in particular osteoarthritis and/or a pain-associated osteoarthritis, may be administered in combination with colchicine.

As used herein, the term "combination", "combined," and related terms refers to the simultaneous or sequential administration of said additional active ingredient with colchicine. For example, a combination may be administered with an additional active ingredient simultaneously or sequentially in separate unit dosage forms or together in a single unit dosage form.

According to one embodiment, hyaluronic acid may be injected into the painful joint, and for example be present in pharmaceutical composition according to the present invention. The use of hyaluronic acid in the treatment of arthritis, such as osteoarthritis, is well known, as visco supplementation. Therefore, according to one embodiment, the present invention provides a pharmaceutical composition comprising controlled release dosage form comprising colchicine according to the present invention further comprising hyaluronic acid.

In some embodiments, the present invention provides a pharmaceutical composition comprising controlled release dosage form comprising colchicine according to the present invention further comprising at least an additional therapeutic agent. Suitable additional active ingredients are described in further detail below.

According to one embodiment, the following active ingredients may be combined, in particular any anti-inflammatory active ingredient:
- (i) a corticosteroid, such as prednisone, prednisolone, methylprednisolone, betamethasone, dexamethasone, triamcinolone acetonide, triamcinolone hexacetonide, budenoside, mometasone, ciclesonide, fluticasone, hydrocortisone, and pharmaceutically acceptable salts thereof, especially at low dose,
- (ii) an NSAID, such as aspirin, diclofenac, aceclofenac, sulindac, ketorolac, ibuprofen, ketoprofen, naproxen, oxaprozine, flubiprofen, indomethacin, proglumetacin, tiaprofenic acid, meloxicam, piroxicam, tenoxicam, etodolac and celecoxib, etoricoxib, parecoxib, rofecoxib, valdecoxib, and pharmaceutically acceptable salts thereof,
- (iii) an antiIL-1β agent such as anakinra, canakinumab, rilonacept, and pharmaceutically acceptable salts thereof,
- (iv) an anti-IL-6 agent such as tocilizumab, siltuximab, sarilumab, and pharmaceutically acceptable salts thereof,
- (v) an anti-TNFα agent, such as adalimumab, etanercept, infliximab, certolizumab, golimumab, and pharmaceutically acceptable salts thereof,
- (vi) an anti-angiogenic agent such as bevacizumab, and pharmaceutically acceptable salts thereof,
- (vii) an anti NGF (Nerve Growth Factors) agent, such as fasinumab, tanezumab, fulranumab, ABT-110, and pharmaceutically acceptable salts thereof,
- (viii) an opioid, such as fentanyl, morphine, buprenorphine, hydromorphone, hydrocodone, oxycodone, meperidine, and pharmaceutically acceptable salts thereof,
- (ix) an inhibitor of class I glucose transporters (e.g. GLUT-1/GLUT-3), such as cytochalasin B, WZB-117, STF-31, BAY-876,
- (x) mixtures thereof.

Said additional active ingredient may be formulated under immediate and/or controlled release dosage forms.

### Administration of the composition

A pharmaceutical composition used in the framework of the present invention can be injected using any of the known methods in the art.

Particularly, said pharmaceutical composition may be administered by means of an injection device suitable for intra-articular injection such as a syringe equipped with a needle 19-29G, preferentially 22-29G, more preferentially 25-29G.

Throughout the description, including the claims, the expression "comprising a" should be understood as being synonymous with "comprising at least one" unless specifically stated otherwise.

The expressions "between... and ..." and "ranging from ... to ..." should be understood to mean that the limits are inclusive, unless specified otherwise.

The following examples and figures are presented by way of non-limiting illustration of the invention.

### EXAMPLES

### Analytical Methods

- Particle **size** was determined using a Malvern Mastersizer MS3000 laser diffraction particle size analyzer. A preparation of 0.1 to 1 mg of microparticles per ml of deionized water was introduced in the apparatus. The measurement was run three times. Each run lasted for 3 seconds, and the suspension was homogenized before each run. The results shown in the examples are the mean of the three runs.
- Microparticle **drug load** of Examples 1 and 2 were determined by dissolving 30 mg of particles with 2,5 mL acetonitrile in a 50 mL volumetric flask. After dissolution, 22,5 mL of methanol is added and mixed under vortexing. The flask was then filled up with water and thoroughly mixed using vortex. The medium was then centrifuged for 30 min at 4000 rpm and the supernatant is filtrated through a 0.45 µm PTFE filter (Acrodisc wwPTFE), discarding the first 2 mL. An aliquot of this solution is then analyzed by HPLC to determine the microparticle drug load.
- **Analysis by HPLC** in Examples 1 and 2 were conducted using a silica-based reversed-phase C8 column [GL Sciences, Inertsil C8-3, 4.6 × 250 mm (5µm)] and a mobile phase [55% methanol, 45% of a 6.8 g/L KH₂PO₄ solution, solution adjusted to pH 5.5 with diluted phosphoric acid] at 1 mL/min flow rate with UV detection at 254 nm.
- For the ***in vitro* release tests** from microparticles of Examples 1, 2 and 3, aliquots of 20 mg microparticles were suspended in 50 mL of phosphate buffer (50 mM, pH 7.4) containing 0.02% sodium azide and maintained at 37°C in a horizontal shaker set at 80 rpm. At different intervals, samples of 3 mL of the media were withdrawn and replaced with fresh medium. The removed sample was centrifuged at 4000 rpm for 3 minutes and the supernatant analyzed by UV at 350 nm against standard.

### Example 1: Sustained-release Colchicine microparticles

A 1 wt% polyvinyl alcohol (PVA, Mowiol 4-88, Sigma-Aldrich) stock solution was prepared by heating 594 g water to 70°C and dispersing therein 6 g of PVA under magnetic stirring until full solubilization. The solution was then allowed to cool down prior to further use.

In a separate container, 0.7 g of colchicine (INDENA, Italy) were solubilized in 2.5 g methylene chloride (Merck) under vortex agitation until complete solubilization. Then, 1,05 g of PLGA 75:25 Resomer RG 752 S [poly(lactic-co-glycolic acid) copolymer 75:25 with inherent viscosity determined by an Ubbelohde capillary viscometer of 0.16-0.24 dl/g; (Evonik Industries AG, Essen, Germany)] were introduced in the mixture and completely dissolved under vortex agitation to form a polymer-drug solution.

5 g of the previously prepared PVA 1 wt% stock solution were then added to the drug-polymer solution over 30 seconds under high shear (Heidolph homogenizer DIAX 900, 11 600 RPM) to form a pre-emulsion.

This pre-emulsion was then slowly poured into a hardening bath containing 500 ml of the PVA 1 wt% stock solution previously prepared.

Agitation was maintained using a double propeller stirrer with a stirring speed set at 250 rpm for 3 hours until methylene chloride is evaporated.

The formed microparticles were centrifuged at 4000 rpm for 3 minutes using a swinging bucket centrifuge apparatus to remove the main portion of the water phase and get a concentrated particle slurry prior separation per 12 µm stainless steel-sieve (4 cm diameter).

The microparticles were then rinsed with 150 ml water and dried overnight in a desiccator over 3Å molecular sieve.

The collected microparticles were then stored at 5°C.

Analytical tests, including the *in vitro* profile, were performed as described in the "Analytical Methods" section above, and are shown in Table 2 below.

**Table 2. In vitro release profile for Colchicine PLGA microparticles**

| Colchicine loading content (wt %) | *In vitro* release in 50 mM Phosphate Buffer, pH 7.4 | Particle size (Dv, µm) |
|---|---|---|
| | n=2 | |
| | (%) | |
| 3.0 | 0.13 day: 16.5 | D10 = 11 µm |
| | 1 day: 23.8 | |
| | 9 days: 58.3 | |
| | 16 days: 59.5 | D90 = 25 µm |
| | 22 days: 60.9 | Mean Particle Size = 19 µm |
| | 30 days: 72.5 | |
| | 37 days: 78.0 | |
| | 44 days: 94.5 | |
| | 51 days: 100.9 | |

In this example, colchicine loaded PLGA microparticles were prepared with a colchicine loading content of 3 wt %, a mean particle size of 19 µm and a drug release maintained over more than 30 days.

### Example 2: Sustained-release Colchicine microparticles

The same Polyvinyl alcohol (PVA) stock solution as in Example 1 was prepared.

In a separate container, 0.7 g of colchicine (INDENA, Italy) were solubilized in 2.5 g methylene chloride (Merck) under vortex agitation until complete solubilization. Then, 1,05 g of PLGA 75:25 Resomer RG 755 S [poly(lactic-co-glycolic acid) copolymer 75:25 with inherent viscosity determined by an Ubbelohde capillary viscometer of 0.5-0.7 dl/g; (Evonik Industries AG, Essen, Germany)] were introduced in the mixture and completely dissolved under vortex agitation to form a polymer-drug solution.

5 g of the previously prepared PVA 1 wt% stock solution were then added to the drug-polymer solution over 30 seconds under high shear (Heidolph homogenizer DIAX 900, 11 600 rpm) to form a pre-emulsion.

This pre-emulsion was then slowly poured into a hardening bath containing 500 ml of the PVA 1 wt% stock solution previously prepared.

Agitation was maintained using a double propeller stirrer with a stirring speed set at 250 RPM for 3 hours until methylene chloride is evaporated.

The formed microparticles were centrifuged at 4000 rpm for 3 minutes using a swinging bucket centrifuge apparatus to remove the main portion of the water phase and get a concentrated particle slurry prior separation per 12 µm stainless steel-sieve (4 cm diameter).

The microparticles were then rinsed with 150 ml water and dried overnight in a desiccator over 3Å molecular sieve.

The collected microparticles were then stored at 5°C.

Analytical tests, including the *in vitro* profile, were performed as described in the "Analytical Methods" section above, and are shown in Table 3 below.

**Table 3. In vitro release profile for Colchicine PLGA microparticles**

| Colchicine loading content (wt %) | *In vitro* release in 50 mM Phosphate Buffer, pH 7.4 | Particle size (Dv, µm) |
|---|---|---|
| | n=2 | |
| | (%) | |
| 6.0 | 1 day: 1.3 | D10 = 16 µm |
| | 8 days: 7.4 | |
| | 15 days: 5.6 | |
| | 22 days: 8.0 | |
| | 29 days: 9.9 | D90 = 60 µm |
| | 36 days: 11.8 | |
| | 50 days: 14.5 | Mean Particle Size = 25 µm |
| | 64 days: 17.9 | |
| | 72 days: 22.7 | |
| | 79 days: 38.9 | |
| | 85 days: 76.7 | |
| | 93 days: 91.4 | |
| | 100 days: 90.9 | |

In this example, colchicine loaded PLGA microparticles were prepared with a colchicine loading content of 6 wt %, a mean particle size of 25 µm and a drug release maintained over at least 100 days.

### Example 3: Sustained Release Colchicine microparticles composition

A Sustained Release colchicine microparticles composition was prepared by blending 0.2 g of particles of example 1 and 0.2 g of particles of example 2.

*In vitro* profile was performed as described in the "Analytical Methods" section above and is shown in Table 4 below.

**Table 4. In vitro release profile for Colchicine PLGA microparticles blend**

| | |
|---|---|
| *In vitro* release in 50 mM Phosphate Buffer, pH 7.4 | 1 day: 10.7 |
| | 8 days: 31.3 |
| | 15 days: 33.2 |
| | 22 days: 37.1 |
| | 30 days: 41.1 |
| n=2 | 38 days: 52.4 |
| (%) | 55 days: 61.0 |
| | 70 days: 81.2 |
| | 90 days: 97.8 |

### Example 4: Pharmaceutical composition

An aqueous injection vehicle was prepared using pyrogen-free excipients and consisting of 1.4% of low viscosity sodium carboxymethyl cellulose (Aqualon CMC 7LF PH BET, Ashland), 0.1% Polysorbate 20 (Acros Organics), 0.13% disodium hydrogen phosphate dihydrate, 0.1% citric acid (Roth) and 0.65% sodium chloride (Roth). Final pH of the solution was adjusted to 7.2 using concentrated sodium hydroxide solution (Roth). The vehicle was then autoclaved at 121°C for 15 minutes (MultiControl 2, CertoClav) and 5 mL aliquots of the solution transferred aseptically into 10 mL vials under a laminar air flow bench.

Aliquots of microparticles as prepared according to example 3 were dispersed in each vial using a vortex mixer. Pharmaceutical compositions under the form of a sterile and injectable dosage form suitable for an intra-articular injection according to the invention may thus be prepared.

These pharmaceutical compositions are suitable for intra-articular injection to treat a joint disease such as osteoarthritis and any other type of chondropathy and/or a pain-associated joint disease, in particular osteoarthritis and/or a pain-associated osteoarthritis.

## Claims

1. A pharmaceutical composition under the form of a sterile and injectable dosage form comprising a controlled release dosage form comprising colchicine, for use in the treatment of a joint disease such as osteoarthritis and any other type of chondropathy and/or a pain-associated joint disease, by intra-articular injection into a joint of said pharmaceutical composition, wherein the time required to release 80% by weight of the colchicine is greater than one month and wherein the colchicine is present in a concentration ranging from 0.003 to 27.5 mg per ml of the sterile and injectable dosage form.

2. The pharmaceutical composition for use according to claim 1, wherein the time required to release 80% by weight of the colchicine lies between one to six months, two to six months or three to six months.

3. The pharmaceutical composition for use according to claim 1 or 2, wherein the controlled release dosage form comprising colchicine is under the form of an in-situ forming depot or microparticles, in particular microparticles comprising a polymeric matrix or multivesicular liposomes.

4. The pharmaceutical composition for use according to anyone of the preceding claims, wherein the controlled release dosage form comprising colchicine is under the form of microparticles, in particular microparticles comprising a polymeric matrix, and wherein said microparticles comprising colchicine have a mean particle size determined by laser light diffraction measurement method equal or greater than 10 µm, and in particular have a mean particle size lower than 100 µm, more particularly lower than 80 µm, and even more particularly lower than 50 µm, for example between 10 and 50 µm or between 10 and 40 µm.

5. The pharmaceutical composition for use according to claim 4, wherein the microparticles are microparticles comprising colchicine and a polymeric matrix, which may be a mixture of microparticles of different nature, wherein the polymeric matrix comprises at least one poly(lactic-co-glycolic acid) copolymer, at least one poly(caprolactone) or at least a mixture of at least one poly(lactic-co-glycolic acid) copolymer and at least one poly(caprolactone).

6. The pharmaceutical composition for use according to anyone of claims 4 to 5, wherein the polymeric matrix further comprises one or more additional polymer(s) or copolymer(s) selected from poly(lactide) distinct from poly(lactic-co-glycolic acid) copolymer, poly(glycolide) distinct from poly(lactic-co-glycolic acid) copolymer, poly(lactide-co-caprolactone), poly(ethylene glycol), poly(ethylene oxide), PLGA-b-PEO-b-PLGA, PLGA-b-PEO, polyhydroxyalkanoates, poly(hydroxybutyrate), poly(trimethylene carbonate), poly(dioxanone), poly(valerolactone), poly(alpha- hydroxy acids), poly(lactones), poly(amino acids), polyanhydrides, poly(orthoester), poly(acetals), polyurethanes, polythioesters, polyphosphoesters, poly(ester-co-amide), poly(vinyl alcohol), PVA-g-PLGA, poly (ether ester) multiblock copolymers, polyvinyl pyrrolidone, poly(methacrylate), PEO-PPO-PEO, gelatin, heparin, chondroitin sulfate; polysaccharides such as alginates, starch, chitosan and dextrans and any combinations thereof, and in particular selected from poly(lactide) and poly(caprolactone).

7. The pharmaceutical composition for use according to anyone of claims 4 to 6, wherein the polymeric matrix comprises at least one poly(lactic-co-glycolic acid) copolymer, at least one poly(caprolactone), or a mixture of at least one poly(lactic-co-glycolic acid) copolymer and at least one poly(caprolactone) in an amount of greater than 70% by weight, with respect to the total weight of the polymeric matrix, in particular greater than 80% by weight, and even more particularly greater than 90% by weight, wherein the poly(lactic-co-glycolic acid) copolymer has in particular a lactic:glycolic molar ratio ranging between 50:50 and 90:10, in particular ranging between 60:40 and 90:10, more particularly ranging between 65:35 and 85:15, for example 75:25, and wherein the poly(lactic-co-glycolic acid) copolymer in particular exhibits an inherent viscosity determined by an Ubbelohde capillary viscometer of 0.1 to 1.7 dl/g, in particular of 0.1 to 1.4 dl/g and even more particularly from 0.1 to 0.9 dl/g.

8. The pharmaceutical composition for use according to anyone of the preceding claims, wherein it is under the form of a sterile and injectable dosage form selected from a solution, a suspension, a solid implant, a semi-solid implant, a powder and an in-situ depot.

9. The pharmaceutical composition for use according to anyone of the preceding claims, wherein the colchicine is present in a concentration ranging from 0.003 to 27.5 mg per ml of the sterile and injectable dosage form, in particular a suspension, in particular from 0.003 to 13.75 mg per ml.

10. The pharmaceutical composition for use according to anyone of the preceding claims in osteoarthritis patients with inflammatory component, inflammatory pain or joint effusion.

11. The pharmaceutical composition for use according to anyone of the preceding claims, wherein the pharmaceutical composition is further **characterized in that** it is effective to maintain systemic concentration of colchicine 24 hours after the intra-articular injection, in particular over 1 month, and even more particularly over at least 3 months, lower than 5 ng/ml, particularly lower than 1 ng/ml, more particularly lower than 0.5 ng/ml, and even more particularly lower than 0.1 ng/ml and to maintain synovial concentration of colchicine after the intra-articular injection, over 1 month, and even more particularly over at least 3 months, greater than 0.5 ng/ml, in particular comprised between 0.5 and 100 ng/ml and more particularly comprised between 0.5 and 50 ng/ml.

12. The pharmaceutical composition for use according to anyone of the preceding claims, wherein it is for use in the treatment of a joint disease, such as osteoarthritis and any other type of chondropathy and/or a pain-associated joint disease, in particular osteoarthritis and/or a pain-associated osteoarthritis in a patient being simultaneously, separately or sequentially treated by at least one of the following active ingredients: atazanavir, clarithromycin, darunavir, ritonavir, indinavir, itraconazole, ketoconazole, lopinavir, nefazodone, nelfinavir, saquinavir, telithromycin, tipranavir, amprenavir, aprepitant, diltiazem, erythromycin, fluconazole, fosamprenavir, verapamil, cyclosporine and ranolazine.

13. A pharmaceutical composition under the form of a sterile and injectable dosage form comprising a controlled release dosage form comprising colchicine, wherein said pharmaceutical composition presents an *in vitro* dissolution profile, wherein less than 25% of colchicine, and more preferentially less than 15% of colchicine, is released within 24 hours, more than 80% of colchicine is released within at least one month, in particular within three months, and even more particularly within six months, and wherein the colchicine is present in a concentration ranging from 0.003 to 27.5 mg per ml of the sterile and injectable dosage form.

14. A formulation under the form of a powder comprising a controlled release dosage form comprising colchicine, under the form of microparticles having a mean particle size determined by laser light diffraction measurement method equal or greater than 10 µm, comprising a polymeric matrix, wherein the polymeric matrix comprises at least one poly(lactic-co-glycolic acid) copolymer, at least one poly(caprolactone), or a mixture of at least one poly(lactic-co-glycolic acid) copolymer and at least one poly(caprolactone) in an amount of greater than 70% by weight, with respect to the total weight of the polymeric matrix, in particular greater than 80% by weight, and even more particularly greater than 90% by weight, wherein the poly(lactic-co-glycolic acid) copolymer has in particular a lactic:glycolic molar ratio ranging between 50:50 and 90:10, in particular ranging between 60:40 and 90:10, more particularly ranging between 65:35 and 85:15, for example 75:25, and wherein the poly(lactic-co-glycolic acid) copolymer in particular exhibits an inherent viscosity determined by an Ubbelohde capillary viscometer of 0.1 to 1.7 dl/g, in particular of 0.1 to 1.4 dl/g and even more particularly from 0.1 to 0.9 dl/g.

15. A pharmaceutical composition under the form of a sterile and injectable dosage form suitable for an intra-articular injection, obtained by mixing a formulation under the form of a powder according to the claim 14, with an aqueous injection vehicle, wherein the pharmaceutical composition optionally comprises an excipient selected from a group consisting of a tonicity-enhancing agent, a wetting-agent, a viscosity-enhancing agent, a density-enhancing agent or a mixture thereof, and wherein said excipient may be present in the aqueous injection vehicle or in the powder.

16. A pharmaceutical composition under the form of a sterile and injectable dosage form suitable for an intra-articular injection, comprising a controlled release dosage form comprising colchicine, under the form of microparticles having a mean particle size determined by laser light diffraction measurement method equal or greater than 10 µm comprising a polymeric matrix, wherein the polymeric matrix comprises at least one poly(lactic-co-glycolic acid) copolymer, at least one poly(caprolactone), or a mixture of at least one poly(lactic-co-glycolic acid) copolymer and at least one poly(caprolactone) in an amount of greater than 70% by weight, with respect to the total weight of the polymeric matrix, in particular greater than 80% by weight, and even more particularly greater than 90% by weight, wherein the poly(lactic-co-glycolic acid) copolymer has in particular a lactic:glycolic molar ratio ranging between 50:50 and 90:10, in particular ranging between 60:40 and 90:10, more particularly ranging between 65:35 and 85:15, for example 75:25, and wherein the poly(lactic-co-glycolic acid) copolymer in particular exhibits an inherent viscosity determined by an Ubbelohde capillary viscometer of 0.1 to 1.7 dl/g, in particular of 0.1 to 1.4 dl/g and even more particularly from 0.1 to 0.9 dl/g.

17. The pharmaceutical composition under the form of a sterile and injectable dosage form according to claim 15 or 16, wherein it is selected from a solution, a suspension, a solid implant, a semi-solid implant, a powder and an in-situ depot, in particular a suspension, and wherein colchicine is present in a concentration ranging from 0.003 to 27.5 mg per ml of the sterile and injectable dosage form, in particular a suspension, in particular from 0.003 to 13.75 mg per ml.

18. A kit or article of manufacture comprising, in separate compartments (i) an aqueous injection vehicle and (ii) a controlled release dosage form comprising colchicine as defined in anyone of claims 3 to 7 or powder as defined in claim 14, wherein the kit or article of manufacture optionally comprises an excipient selected from a group consisting of a tonicity-enhancing agent, a wetting-agent, a viscosity-enhancing agent, a density-enhancing agent or a mixture thereof, for preparing a pharmaceutical composition suitable for an intra-articular injection.

19. The kit or article of manufacture according to claim 18, wherein it is under the form of a vial and a prefilled syringe or of a medical device or is under the form of two separated vials.
